# EUROPÄISCHE PATENTSCHRIFT

(11) **EP 3 441 455 B1**
(45) Veröffentlichungstag und Bekanntmachung des Hinweises auf die Patenterteilung: **01.07.2020**
(21) Anmeldenummer: 18401072.6
(22) Anmeldetag: 04.08.2018
(51) Int. Cl.: C12M 1/107, C02F 1/34, C12M 1/00, C12M 1/42, C12M 1/34

(54) **VORRICHTUNG ZUM AUFSCHLUSS VON UND ZUR GEWINNUNG EINER AMMONIUMSULFATLÖSUNG AUS ORGANISCHEN SUBSTRATEN**
APPARATUS FOR DIGESTING AND RECOVERING AN AMMONIUM SULFATE SOLUTION FROM ORGANIC SUBSTRATES
APPAREIL POUR LA DIGESTION ET LA RÉCUPÉRATION D'UNE SOLUTION DE SULFATE D'AMMONIUM À PARTIR DE SUBSTRATS ORGANIQUES

(30) Priorität: 11.08.2017 DE 102017118344
(43) Veröffentlichungstag der Anmeldung: 13.02.2019
(73) Patentinhaber: Biogastechnik Süd GmbH, 88316 Isny im Allgäu (DE)
(72) Erfinder: Maier, Clemens, 88316 Isny-Sommersbach (DE); Maier, Gregor, 88316 Isny-Sommersbach (DE)
(74) Vertreter: Höchtl, Walter

(56) Entgegenhaltungen:
- EP-A1- 1 717 209
- EP-A1- 3 015 434
- EP-B1- 3 066 187
- DE-A1-102005 030 895
- DE-A1-102012 011 555
- DE-A1-102013 000 977
- DE-A1-102016 012 373

## Beschreibung

Die Erfindung bezieht sich auf eine Vorrichtung zum Aufschluss von und zur Gewinnung einer Ammoniumsulfatlösung aus organischen Substraten. Organische Substrate werden auch als Biomasse bezeichnet - wie beispielsweise Klärschlamm oder Biomasse aus nachwachsenden Rohstoffen (NaWaRo). Die organischen Substrate können aus einem Lager für die Biomasse, einer sogenannten Vorgrube, einem Vorgärer, einem Fermenter, z. Bsp. aus einem Biogas - Fermenter stammen; oder es kann sich z. Bsp. um Gülle aus einem landwirtschaftlichen Betrieb oder pumpfähige Biomasse handeln. Prinzipiell kann jedes organische Substrat mit der erfindungsgemäßen Vorrichtung aufgeschlossen werden.

Zum Aufschluss des jeweiligen organischen Substrates verfügt das Aufschlussmodul über eine Vorrichtung zur Erzeugung von Kavitation. Unter "Aufschluss" wird daher im Sinne dieser Anmeldung die Behandlung von organischem Substrat mit einer Vorrichtung zur Erzeugung von Kavitation verstanden. Der Kavitations-Effekt sowie entsprechende Vorrichtungen sind beispielsweise aus der europäischen Patentanmeldung EP3015434A1 und den darin angeführten Dokumenten bekannt: Ein Teil der Zellen der Biomasse wird durch Kavitation aufgeschlossen und dabei fließfähiger. Dieses fließfähigere Material ist danach z.B. in den Fermentern oder Nachgärem einer Biogasanlage von den Mikroorganismen besser umsetzbar. Das heißt, die Faulung wird optimiert, so dass bis zu 50 % mehr Biogas und dementsprechend weniger Restsubstrat als Abfallprodukt entsteht. Als weitere positive Effekte werden die Einsparung von Faulraumvolumen und eine verbesserte Entwässerbarkeit der Biomasse genannt (siehe http://www.sonotronic.de/technologien/ultraschall/ultraschallbehandlung-von-biofeststoffen). Die von der Firma Sonotronic beschriebene Technology erzeugt die Kavitation mit Ultraschallsensoren, sogenannten Sonotroden, die direkt in der Biomasse angeordnet sind.

Die mit Ultraschall betriebenen Kavitations - Vorrichtungen respektive Aufschlussmodule arbeiten im Gegensatz zur vorliegenden Erfindung weniger effizient. Dies liegt vor allen daran, dass die Ultraschallsensoren (Sonotroden) direkt im Substrat angeordnet sind und die Ausbreitung des Schalls im Substrat von der Leistung dieser Sensoren abhängig ist. Nimmt man leistungsstarke Sonotroden, so ist die Gefahr der (Selbst-)Beschädigung durch die von ihnen erzeugte Kavitation sehr groß und die Standzeiten der Sonotroden sind entsprechend gering.

DE 10 2016 012373 offenbart eine Vorrichtung zum Aufschluss von biologischen Materials mit einem Behandlungsraum zur Aufnahme des biologischen Materials als Dispersion und mindestens einem Schwingsystem mit definierter Resonanzfrequenz. Es handelt sich beispielsweise um vertikale mechanische Schwingungen, die in der Dispersion Kavitationen erzeugen. Die Vorrichtung umfasst einen Behälter mit einer Einführöffnung und einer Austrittsöffnung zum kontinuierlichem Betrieb. Der Überstand über der Dispersion kann mittels Vakuumpumpe evakuiert werden.

Die vorliegende Erfindung sieht daher zur Erzeugung von Kavitation elektromagnetische Vorrichtungen bzw. elektrisch betriebene Vibratoren oder elektrische bzw. elektromechanische Aktuatoren vor, die außerhalb des Substrates angeordnet sind und die die Schwingungen bzw. Vibration in das Substrat mittelbar über den unter Vakuum stehenden Behälter oder über andere Vorrichtungen einbringen und so im Substrat derartige vertikale Schwingungen anregen, das Kavitation entsteht.

Das Aufschlussmodul besteht unter anderem aus einem luftdichten Behälter zur Aufnahme des Substrates, der nur teilweise mit dem Substrat gefüllt ist. Im substratfreien Raum oberhalb des Substratspiegels befindet sich ein Dampfauslass über den der entstehende Brüden mittels einer Vakuumpumpe abgesaugt werden kann, wobei das entstehende Vakuum einerseits den Aufschlussprozess, i. e. die Kavitation, deutlich verstärkt und andrerseits der Brüden mit mehr Ammoniak angereichert wird, als es bei herkömmlicher Vergärung der Fall ist.

Der Nachteil der in der EP3015434A1 beschriebenen Vorrichtung besteht allerdings darin, dass herkömmliche Vakuumpumpen nicht in der Lage sind, ausreichend Brüden abzusaugen, so dass ein gewünschter Unterdruck im Aufschlussmodul entsteht.

Aufgabe der Erfindung ist es daher, eine Vorrichtung zur Verfügung zu stellen, mit der der Aufschluss von organischen Substraten mit einer elektromagnetischen Aufschlussvorrichtung optimiert wird.

Diese Aufgabe wird durch die Merkmale des Hauptanspruches gelöst. Dabei ist der Vakuumpumpe ein Kondensator vorgeordnet, in dem der Brüden abgekühlt und kondensiert wird, so dass die Vakuumpumpe ein geringeres und dichteres Volumen zu Pumpen hat, wobei an dem Dampfauslass (DA) eine Vakuumpumpe (V) angeschlossen ist, und wobei zwischen der Vakuumpumpe (V) und dem Dampfauslass (DA) ein Brüdenwäscher (W) und ein Kondensator (K) angeordnet ist.

Nach einer alternativen Ausbildung der Erfindung übernimmt die Kondensation des Dampfes / des Brüden bis zu einer bestimmten Dampfmenge auch eine wassergekühlte Vakuumpumpe, wobei nach dem Dampfauslass (DA) ein Brüdenwäscher (W) und eine wassergekühlte Vakuumpumpe (V) angeschlossen sind.

Durch diese optimierte Vakuumabsaugung wird sowohl der Fermentationsprozess zur Erzeugung von Biogas mittels der Kavitation verbessert, als auch die Sättigung des Brüden mit Ammoniak verstärkt, was sich wiederum positiv auf die Biogasproduktion auswirkt, da ein zu hoher Ammoniak Anteil die Biogasproduktion bremsen kann. Zusätzlich steigt der Gehalt an Ammoniak im Brüden, so dass eine verstärkte Produktion von Dünger in Form einer Ammoniumsulfatlösung (ASL) ermöglicht wird.

Damit wird das Ammoniak in Form einer Ammoniumsulfatlösung (ASL) aus dem Brüden ausgewaschen und das getrennte ASL kann als Dünger weiter verwendet werden. Das verbleibende Wasser kann z. Bsp. in die Kanalisation eingeleitet werden.

Eine weitere vorteilhafte Ausbildung der Erfindung besteht darin, dass nach dem Dampfauslass ein Abscheider vorgesehen ist. Da es wegen der Kavitation kaum vermeidbar ist, dass auch Substratbestandteile und / oder Schaum über den Dampfauslass mit abgesaugt werden, ist ein Abscheider sinnvoll, der diese Verunreinigungen von dem Brüden trennt.

Schließlich kann es - je nach Substrat - vorteilhaft sein, am Substratzufluss (SZ) einen Zerkleinerer (Z) vorzusehen, der die Substrat - Konsistenz für den Aufschlussprozess optimiert.

Mit einer erfindungsgemäßen Vorrichtung zur Gewinnung einer Ammoniumsulfatlösung, die eine Vakuumpumpe, einen Kondensator und ein Brüdenwäscher umfasst, kann der in den Verdampfern (AM1, AM2, VD) einer Biogasanlage entstehenden Brüden zentral abgesaugt werden.

Es ist weiter vorteilhaft, vor dem Aufschlussmodul einen Entgasungsbehälter anzubringen mit dem eine Entgasung des Substrates durchgeführt werden kann, bevor das Substrat in das Aufschlussmodul gelangt. Das im Substrat noch enthaltene Kohlendioxid würde sonst durch die Kavitation im Aufschlussmodul frei und würde dort eine starke Schaumbildung verursachen, was die Kavitationswirkung im Aufschlussmodul stark einschränken würde

Ein Ausführungsbeispiel der Erfindung wird anhand der Zeichnungen beschrieben. Dabei zeigt:
- Figur 1: ein Aufschlussmodul AM mit Vakuumabsaugung und Brüdenwäscher
- Figur 2: eine schematische Darstellung der zentralen Absaugung des Brüden in einer Biogasanlage
- Figur 3: das Aufschlussmodul AM gemäß Figur 1 mit einem Entgasungsbehälter EB

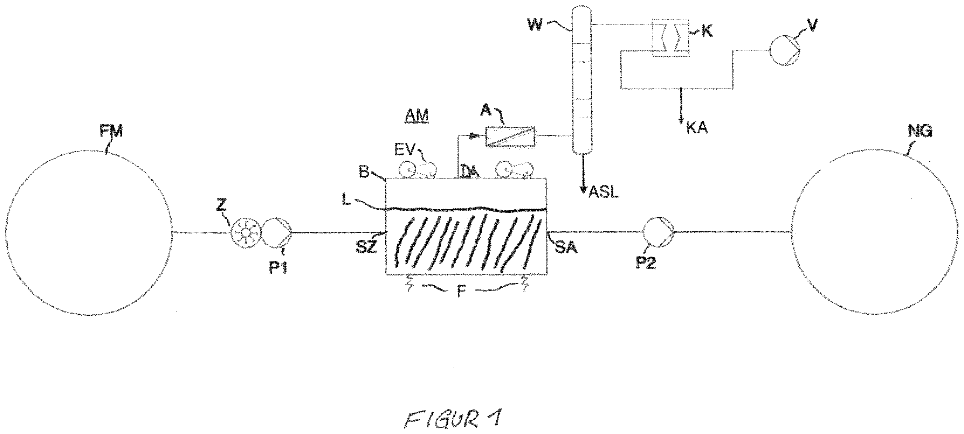

Figur 1 zeigt von links nach rechts einen Fermenter FM, beispielsweise von einer Biogasanlage, aus dem das organische Substrat über einen Zerkleinerer Z und eine letzterem nachgeordnete Pumpe P1 über den Substratzufluss SZ in das Aufschlussmodul AM gespeist wird. Der Zerkleinerer ist nur bei entsprechender Substrat - Konsistenz notwendig. Wird beispielsweise Gülle verarbeitet, ist er nicht notwendigerweise vorzusehen.

Das Aufschlussmodul AM besteht unter anderem aus dem luftdichten Behälter B, der nur bis zum Level L mit Substrat gefüllt ist. Im Hohlraum oberhalb des Substratfüllstandes L wird der Brüden über den Dampfauslass DA und die Vakuumpumpe V abgesaugt.

Am Behälter B des Aufschlussmoduls AM sind zwei elektrische Aktuatoren, hier zwei elektromagnetische Vorrichtungen EV, angebracht. Diese bringen den Behälter B in vertikale Schwingungen, so dass im Substrat Kavitationsblasen entstehen. Der Behälter ruht auf vier gefederten Standbeinen F.

Das Substrat wird über den Substratabfluss SA aus dem Behälter B mittels der Pumpe P2 ausgeleitet und in den Nachgärer NG verbracht, wo durch das aufgeschlossene Substrat die Biogasproduktion optimiert wird. Substratzu- und -abfluss von und zum Aufschlussmodul AM können auch kontinuierlich erfolgen.

Im Ausführungsbeispiel kommt das Substrat mit einer Temperatur von ca. 50 °C aus dem Fermenter FM. Durch das Vakuum im luftdichten Behälter B - von beispielsweise ca. 80mbar absolut, beginnt das Substrat schon bei ca. 42GradCelsius zu verdampfen. Dadurch werden ca. 9kWh pro m³ Substrat Energie in Form von Wasserdampf mit Ammoniak frei. Dieser Brüden kann nicht mehr von herkömmlichen bzw. ökonomisch sinnvollen Vakuumpumpen abgesaugt werden. Er muss vor der Vakuumpumpe erst wieder gekühlt werden. Deshalb ist der Vakuumpumpe V ein Kondensator K vorgelagert, in dem der Brüden kondensiert und abgekühlt wird. Das kondensierte Wasser wird über die Leitung KA ausgebracht.

Bis zu einer bestimmten Dampfmenge kann die Kondensation des Dampfes / des Brüden auch eine wassergekühlte Vakuumpumpe übernehmen wobei dann allerdings das Ammoniak im Brüden verbleibt, das dann, wenn es als Dünger gewonnen werden soll über eine Umkehrosmose geleitet werden muss. Als Vakuumpumpe kann dabei beispielsweise eine Flüssigkeitsringpumpe, auch Wasserringpumpe genannt, verwendet werden, bei der das Dichtwasser zusätzlich gekühlt wird.

Mit einem dem Kondensator K vorgeschalteten Brüdenwäscher W wird das Ammoniak aus dem Brüden ausgewaschen und kann als Ammoniumsulfatlösung (ASL) Dünger weiter verwendet werden.

Schließlich befindet sich zwischen dem Dampfauslass DA und dem Brüdenwäscher W noch ein Abscheider A mit dem eventuelle Feststoffe aus dem Brüden ausgeschieden werden können.

Die Größe des Behälters B ist abhängig von der Leistung der elektrischen Aktuatoren. Eine Behältergröße von bis zu 200Litern ist derzeit denkbar.

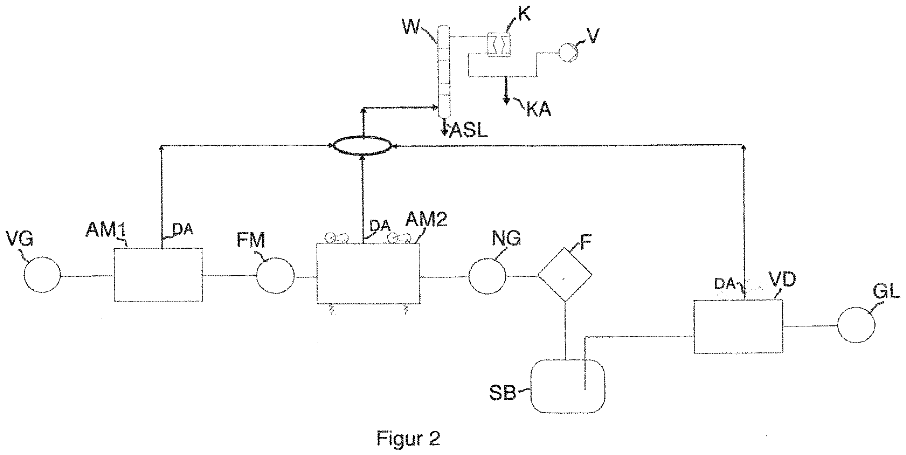

Figur 2 zeigt eine schematische Darstellung der zentralen Absaugung des Brüden in einer Biogasanlage. Zwischen Vorgrube VG und Fermenter FM befindet sich ein erstes Aufschlussmodul AM1. Zwischen dem Fermenter FM und dem Nachgärer NG befindet sich ein zweites Aufschlussmodul AM2. Nach dem Nachgärer NG, der Fest-Flüßig Trennvorrichtung F und dem Behälter für den Trennschlamm SB befindet sich der Verdampfer VD. Alle Dampfauslasse DA der Brüden bildenden Vorrichtungen AM1, AM2, VD respektive Behälter in denen Brüden entsteht, sind an den Brüdenwäscher W geführt und werden zentral von der Vakuumpumpe abgesaugt. Der Vakuumpumpe ist, wie bereits aus Figur 1 bekannt, wieder ein Kondensator K vorgeschaltet. Die Ableitungen für das Kondensat und für die Ammoniumsulfatlösung (ASL) sind ebenfalls aus Figur 1 bekannt. Die Aufschlussmodule AM1, AM2 können solche mit Kavitation oder herkömmliche Verdampfer sein.

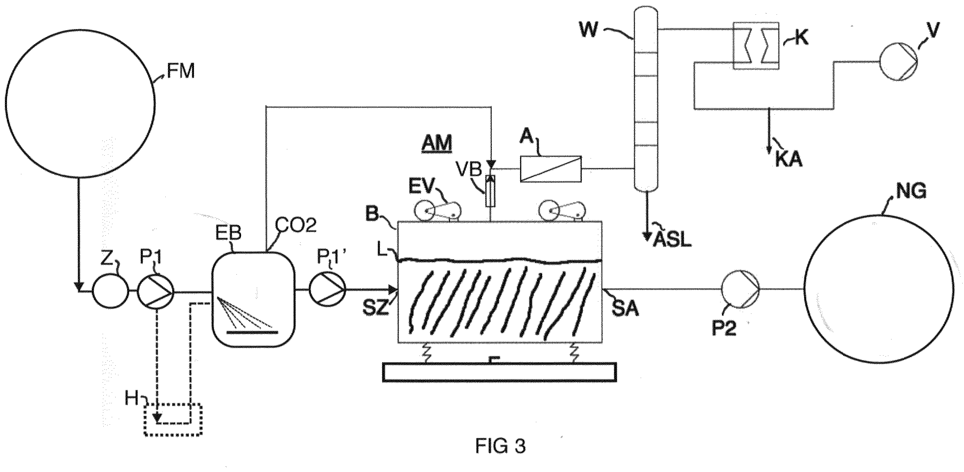

Figur 3 zeigt eine Ausbildung der im wesentlichen aus Figur 1 bekannten Vorrichtung. Dabei ist dem Aufschlussmodul AM ein Entgasungsbehälter EB vorgeschaltet. Zwischen dem Entgasungsbehälter EB und dem Aufschlussmodule ist eine weitere Pumpe P1' vorgesehen. Das Substrat wird durch hier nicht dargestellte Sprühvorrichtungen in den Entgasungsbehälter EB eingesprüht oder eingepumpt, wobei es vorteilhaft auf einen Drehteller T nach Art einer Zentrifuge gelangt. Dadurch wird das im Substrat gebundene Kohlendioxid bereits im Entgasungsbehälter EB frei und gelangt von dort über den Ausgang CO₂ an den Abscheider A. Im Entgasungsbehälter EB herrscht ein höherer Unterdruck (z. Bsp. 0,9bar) als im Aufschlussmodul AM (z. Bsp. 0,8bar), so dass im Aufschlussmodul AM kein oder kaum noch Kohlendioxid frei wird und die Schaumbildung minimiert wird. Der Unterdruck im Entgasungsbehälter wird über die Pumpe P1' und der Unterdruck im Behälter B über das Ventil VB geregelt. Wie durch die gestrichelten Linien zwischen Pumpe P1 und Entgasungsbehälter EB angedeutet, kann alternativ dazwischen eine Heizvorrichtung H vorgesehen werden. Durch die Aufheizung des Substrates mittels der Heizvorrichtung H wird mehr Dampf erzeugt und dadurch die Ammoniakausbeute erhöht.

Das in Figur 3 beschriebene Ausführungsbeispiel kann selbstverständlich auch im Ausführungsbeispiel gemäß Figur 2 angewandt werden.

## Patentansprüche

1. Vorrichtung zum Aufschluss von und zur Gewinnung einer Ammoniumsulfatlösung aus organischen Substraten mit
einem Aufschlussmodul (AM), das
einen luftdichten Behälter (B) mit einem Substratzufluss (SZ), einem Substratabfluss (SA) aufweist, wobei das Aufschlussmodul (AM) mit elektrischen Aktuatoren (EV) zur Erzeugung einer vertikalen Schwingung im Substrat versehen ist,
**dadurch gekennzeichnet, dass**
der Behälter einen Dampfauslass (DA) aufweist, wobei an dem Dampfauslass (DA) eine Vakuumpumpe (V), angeschlossen ist und wobei zwischen der Vakuumpumpe (V) und dem Dampfauslass (DA) ein Brüdenwäscher (W) und ein Kondensator (K) angeordnet ist.

2. Vorrichtung zum Aufschluss von und zur Gewinnung einer Ammoniumsulfatlösung aus organischen Substraten mit
einem Aufschlussmodul (AM), das
einen luftdichten Behälter (B) mit einem Substratzufluss (SZ), einem Substratabfluss (SA) aufweist, wobei das Aufschlussmodul (AM) mit elektrischen Aktuatoren (EV) zur Erzeugung einer vertikalen Schwingung im Substrat versehen ist,
**dadurch gekennzeichnet, dass**
der Behälter einen Dampfauslass (DA) aufweist, wobei nach dem Dampfauslass (DA) ein Brüdenwäscher (W) und eine wassergekühlte Vakuumpumpe (V), angeschlossen sind.

3. Vorrichtung nach einem der vorstehenden Ansprüche, **dadurch gekennzeichnet, dass** nach dem Dampfauslass (DA) ein Abscheider (A) angeordnet ist.

4. Vorrichtung nach einem der vorstehenden Ansprüche, **dadurch gekennzeichnet, dass**, am Substratzufluss (SZ) ein Zerkleinerer (Z) angeordnet ist.

5. Vorrichtung nach einem der vorstehenden Ansprüche, **dadurch gekennzeichnet, dass** dem Aufschlussmodul (AM) ein Entgasungsbehälter (EB) vorgeschaltet ist.

6. Vorrichtung nach Anspruch 5, **dadurch gekennzeichnet, dass** dem Entgasungsbehälter (EB) eine Heizvorrichtung (H) vorgeschaltet ist.

7. Vorrichtung zur Gewinnung einer Ammoniumsulfatlösung aus einer Biogasanlage mit mehreren Verdampfern mit mindestens einer Vorrichtung nach einem der vorstehenden Ansprüche, wobei die Brüden aller Dampfauslässe der Verdampfer (AM1, AM2, VD) über eine einzige Vakuumpumpe (V) abgesaugt werden, wobei zwischen der Vakuumpumpe (V) und dem Dampfatislass (DA) ein Brüdenwäscher (W) und ein Kondensator (K) angeordnet sind.

## Claims

1. An apparatus for digesting and recovering an ammonium sulphate solution from organic substrates with a digestion module (AM) comprising an airtight container (B) with a substrate inflow (SZ), a substrate outflow (SA), wherein the digestion module (AM) is provided with electric actuators (EV) for generating a vertical vibration in the substrate,
**characterised in that**
the container comprises a steam outlet (DA), wherein a vacuum pump (V) is connected to the steam outlet (DA), and wherein an exhaust vapours scrubber (W) and a condenser (K) is arranged between the vacuum pump (V) and the steam outlet (DA).

2. An Apparatus for digesting and recovering an ammonium sulphate solution from organic substrates with a digestion module (AM) comprising an airtight container (B) with a substrate inflow (SZ), a substrate outflow (SA), wherein the digestion module (AM) is provided with electric actuators (EV) for generating a vertical vibration in the substrate,
**characterised in that**
the container comprises a steam outlet (DA), wherein downstream of the steam outlet (DA) an exhaust vapours scrubber (W) and a water-cooled vacuum pump (V) are connected.

3. The apparatus according to any one of the preceding claims, **characterised in that** a separator (A) is arranged downstream of the steam outlet (DA).

4. The apparatus according to any one of the preceding claims, **characterised in that**
a crusher (Z) is arranged on the substrate inflow (SZ).

5. The apparatus according to any one of the preceding claims, **characterised in that**
a degassing container (EB) is connected upstream of the digestion module (AM).

6. The apparatus according to claim 5,
**characterised in that**
a heating device (H) is connected upstream of the degassing container (EB).

7. An apparatus for digesting an ammonium sulphate solution from a biogas facility with several evaporators with at least one device according to any one of the preceding claims, wherein the exhaust vapours of all steam outlets (DA) of the evaporators (AM1, AM2, VD) are extracted by one single vacuum pump (V), wherein an exhaust vapours scrubber (W) and a condenser (K) are arranged between the vacuum pump (V) and the steam outlet (DA).

## Revendications

1. Appareil pour la digestion et la récupération d'une solution de sulfate d'ammonium à partir de substrats organiques avec un module de digestion (AM) comportant un récipient étanche à l'air (B) avec une entrée de substrat (SZ), une sortie de substrat (SA), dans lequel le module de digestion (AM) est muni d'actionneurs électriques (EV) pour produire une vibration verticale dans le substrat.
**caractérisé en ce que**
le récipient comporte une sortie de vapeur (DA), dans lequel une pompe à vide (V) est raccordée à la sortie de vapeur (DA) et dans lequel un laveur de vapeurs (W) et un condensateur (K) est agencé entre la pompe à vide (V) et la sortie de vapeur (DA).

2. Appareil pour la digestion et la récupération d'une solution de sulfate d'ammonium à partir de substrats organiques avec un module de digestion (AM) comportant un récipient étanche à l'air (B) avec une entrée de substrat (SZ), une sortie de substrat (SA), dans lequel le module de digestion (AM) est muni d'actionneurs électriques (EV) pour produire une vibration verticale dans le substrat,
**caractérisé en ce que**
le récipient comporte une sortie de vapeur (DA), dans lequel un laveur de vapeurs (W) et une pompe à vide (V) refroidie à l'eau sont raccordés en aval de la sortie de vapeur (DA).

3. Appareil selon l'une quelconque des revendications précédentes, **caractérisé en ce que**
un séparateur (A) est agencé en aval de la sortie de vapeur (DA).

4. Appareil selon l'une quelconque des revendications précédentes, **caractérisé en ce que**
un concasseur (Z) est agencé à l'entrée de substrat (SZ).

5. Appareil selon l'une quelconque des revendications précédentes, **caractérisé en ce que**
un récipient de dégazage (EB) est agencé en amont du module de digestion (AM).

6. Appareil selon la revendication 5,
**caractérisé en ce que**
un dispositif de chauffage (H) est agencé en amont du récipient de dégazage (EB).

7. Appareil pour la digestion d'une solution de sulfate d'ammonium à partir d'une installation de biogaz avec plusieurs évaporateurs avec au moins un appareil selon l'une quelconque des revendications précédentes, dans lequel les vapeurs de toutes les sorties de vapeur des évaporateurs (AM1, AM2, VD) sont évacuées par une seule pompe à vide (V), dans lequel un laveur de vapeurs (W) et un condensateur (K) sont agencés entre la pompe à vide (V) et la sortie de vapeur (DA).
